(19) Europäisches Patentamt
European Patent Office
Office européen des brevets

(11) EP 2 467 030 B1

# (12) EUROPEAN PATENT SPECIFICATION

(45) Date of publication and mention of the grant of the patent:
**03.07.2013 Bulletin 2013/27**

(21) Application number: **10747020.5**

(22) Date of filing: **20.08.2010**

(51) Int Cl.:
***A23L 1/226*** *(2006.01)* ***C07C 69/16*** *(2006.01)*
***A23L 1/22*** *(2006.01)* ***C07C 69/007*** *(2006.01)*

(86) International application number:
**PCT/EP2010/062160**

(87) International publication number:
**WO 2011/020908 (24.02.2011 Gazette 2011/08)**

## (54) ORGANIC COMPOUNDS ENHANCING THE UMAMI TASTE

ORGANISCHE VERBINDUNGEN, WELCHE DEN UMAMI GESCHMACK VERSTÄRKEN

COMPOSÉS ORGANIQUES AMELIORANT LE GOUT UMAMI

(84) Designated Contracting States:
**AL AT BE BG CH CY CZ DE DK EE ES FI FR GB GR HR HU IE IS IT LI LT LU LV MC MK MT NL NO PL PT RO SE SI SK SM TR**

(30) Priority: **20.08.2009 GB 0914574**

(43) Date of publication of application:
**27.06.2012 Bulletin 2012/26**

(73) Proprietor: **Givaudan SA**
**1214 Vernier (CH)**

(72) Inventors:
- **HOFMANN, Thomas, Frank**
  **85375 Neufahrn (DE)**
- **DEGENHARDT, Andreas**
  **85356 Freising (DE)**

(74) Representative: **Givaudan Patents**
**Givaudan Schweiz AG**
**Ueberlandstrasse 138**
**8600 Dübendorf (CH)**

(56) References cited:
**WO-A1-2007/040399**
**US-A1- 2004 202 619**
**US-A1- 2010 034 944**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

EP 2 467 030 B1

## Description

This invention relates to compounds that are useful in the enhancement of umami taste and savoury flavour, and to their use in compositions and consumable products that contain umami tastants.
Umami is one of the basic tastes and has been described as a savoury or meaty flavour. It is an attribute of many foods, in particularly savoury foods.
Umami taste and savoury flavour is elicited by the salts of glutamic acid, particularly monosodium glutamate (MSG), and these compounds may therefore be used to modify the umami taste and savoury flavour of consumable products. However, some consumers are believed to be adversely affected by glutamate salts, in particularly MSG, and consequently there is a need for compounds that are not based on glutamate to replace or reduce reliance on such compounds for modifying the umami taste and savoury flavour of consumable products.
It is known in the art that the umami taste and savoury flavour of a consumable product can be enhanced by the addition of the naturally-occurring purine nucleotides inosine monophosphate (IMP) and guanosine monophosphate (GMP). However, these compounds are difficult and expensive to produce, thus limiting their use in the industry.
There remains a need for compounds that are useful in the enhancement of umami taste and savoury flavour which do not suffer from the drawbacks of the prior art.
Through the provision of enhancing compounds that are non-glutamate in nature, reliance on compounds, such as MSG, for modifying the umami taste and savoury flavour of compositions and consumable products may be reduced.
The present invention also relates to the use of compounds of formula (I) defined hereinbelow to enhance the umami taste and savoury flavour of consumable products and compositions comprising at least one umami tastant.
The present invention also relates to methods of enhancing the umami taste and savoury flavour of compositions and consumable products comprising at least one umami tastant, by adding to said consumable products and compositions at least one compound of formula (I).
The present invention also relates to compositions and consumable products, comprising at least one umami tastant, and at least one compound of formula (I).
The present invention also relates to novel compounds of formula (I).
The term "umami tastant", as used herein, refers to any compound or ingredient able to elicite an umami taste, non limiting examples of which are provided hereinbelow.
The term "umami taste and savoury flavour enhancer", as used herein, refers to any compound or ingredient, that may or may not elicit an umami taste or savoury flavour itself, that when used in a composition comprising at least one umami tastant results in an increase in the overall umami taste and savoury flavour perception.
The present invention can be understood more readily by reference to the following detailed description and to the examples included herein.
In a first aspect of the present invention there is provided a method of enhancing the umami taste and savoury flavour of a composition, comprising at least one umami tastant, by adding to the aforementioned composition at least one compound of formula (I)

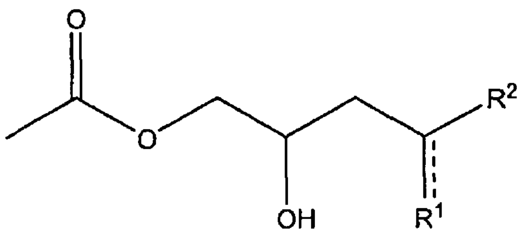

(I)

wherein $R^1$ is selected from O and OH, the dotted line representing a bond present when $R^1$ is O,
$R^2$ is a hydrocarbon residue having 6 to 22 carbon atoms, comprising from 0-4 unsaturated carbon-carbon bonds.
In another particular embodiment compounds of formula (I) are selected from the group consisting of:

1-acetoxy-2-hydroxy-4-oxo-n-heneicosa-5,12,15-trien; 1-acetoxy-2,4-dihydroxy-n-heneicosa-12,15-dien; 1-acetoxy-2,4-dihydroxy-n-heptadeca-16-yne; 1-acetoxy-2-hydroxy-4-keto-n-octadeca-12-en; 1-acetoxy-2,4-dihydroxy-n-heptadeca-16-en; 1-acetoxy-2-hydroxy-4-oxo-n-heneicosa-12,15-dien; 1-acetoxy-2-hydroxy-4-keto-n-heptadeca-16-en; 1-acetoxy-2-hydroxy-4-keto-n-heptadecane.

The compounds according to the present invention may comprise at least one chiral centre, and as such may exist as a mixture of stereoisomers. If it is desired to prepare individual stereoisomers, this may be achieved according to methodology known in the art, e.g. preparative HPLC and GC or by stereoselective syntheses.

In the present invention the use of the term a compound of formula (I) may refer to both a racemic mixture or the individually isolated isomers.

The compounds of formula (I) may be added into a composition in any suitable form for example neat form, or in a solvent, or in a modified form for example they may first be entraped with an entrapment material such as polymers, capsules, microcapsules, nanocapsules, liposomes, precursors, film formers, absorbants such as by using carbon zeolites, cyclic oligosaccharides and mixtures thereof, or they may be chemically bound to substrates which are adapted to release the compounds of formula (I) upon application of an exogenous stimulus such as light, enzymes, or the like.

In another aspect of the present invention there is provided the use of a compound of formula (I) as an umami taste and savoury flavour enhancer.

In a further aspect of the present invention there is provided a composition comprising at least one umami tastant and at least one compound of formula (I).

Compositions according to the present invention comprise at least one umami tastant and at least one compound of formula (I). Additionally the compositions may comprise other ingredients such as other known umami taste or savoury flavour enhancers, and other flavourant ingredients.

Examples of said umami tastants and/or said umami taste or savoury flavour enhancers include, but are not limited to: L-Glu (glutamic acid, glutamate, for example in the form of its salts such as monosodium glutamate, monopotassium glutamate, monoammonium glutamate, calcium diglutamate, magnesium diglutamate), L-Asp (L-asparagine, or a salt thereof), 5'-ribonucleotides or their salts including, without limitation, calcium 5'-ribonucleotides, disodium 5'-ribonucleotides, and dipotassium 5'-ribonucleotides (e.g. inosinic acid, guanylic acid, adenosinic acid, inosinates, guanylates, and adenylates, including guanosine 5'-monophosphate, inosine 5'-monophosphate, and 5'-adenylate and their salts such as disodium guanylate, disodium inosinate, disodium adenylate; dipotassium guanylate, dipotassium inosinate, dipotassium adenylate, calcium guanylate, calcium inosinate, calcium adenylate), maltol, ethyl maltol, glycine, L-leucine, autolyzed or hydrolyzed proteins (e.g. autolyzed yeast, hydrolyzed yeast, hydrolyzed vegetable proteins), Koji-Aji (a nucleotide-rich yeast extract, with fermented wheat gluten and maltodextrin also containing glutamates produced by Ajinomoto Food Ingredients), and natural preparations or extracts containing one or more of the above, for example including extracts, purees or concentrates of vegetables (including mushrooms, shiitake, soy, tomato, potato, whey, kelp/seaweeds), cereals, meat, fish (e.g. shellfish, masago), milk, cheese, and egg yolks, derived from the relevant ingredient in fresh or in fermented, partially or fully hydrolyzed form (e.g. various hydrolysed proteins).

Examples of said other flavourant ingredients include, but are not limited to, natural flavours, artificial flavours, spices, seasonings, and the like. Exemplary flavouring ingredients include synthetic flavour oils and flavouring aromatics and/or oils, oleoresins, essences, distillates, and extracts derived from plants, leaves, flowers, fruits, and so forth, and combinations comprising at least one of the foregoing.

Further examples of other flavourant ingredients can be found in "Chemicals Used in Food Processing", publication 1274, pages 63-258, by the National Academy of Sciences.

Compounds of formula (I) can be used in compositions, according to the present invention, in conjunction with one or more ingredients or excipients conventionally used in flavour compositions, for example carrier materials and other auxiliary agents commonly used in the art. Suitable excipients for flavour compositions are well known in the art, non limiting examples include, solvents (including water, alcohol, ethanol, oils, fats, vegetable oil, and miglyol), binders, diluents, disintegranting agents, lubricants, flavouring agents, coloring agents,_preservatives, antioxidants, emulsifiers, stabilisers, flavour-enhancers, anti-caking agents, and the like.

Examples of such carriers or diluents for flavour compositions may be found in for example, "Perfume and Flavour Materials of Natural Origin", S. Arctander, Ed., Elizabeth, N.J., 1960; in "Perfume and Flavour Chemicals", S. Arctander, Ed., Vol. I & II, Allured Publishing Corporation, Carol Stream, USA, 1994; in "Flavourings", E. Ziegler and H. Ziegler (ed.), Wiley-VCH Weinheim, 1998, and "CTFA Cosmetic Ingredient Handbook", J.M. Nikitakis (ed.), 1st ed.

Other suitable and desirable ingredients of flavour compositions are described in standard texts, such as "Handbook of Industrial Chemical Additives", ed. M. and I. Ash, 2nd Ed., (Synapse 2000).

The compounds of formula (I) are non glutmate in nature and thus may be used to completely substitute or partially substitute MSG in a composition.

In a particular embodiment compositions of the present invention contain salts of glutamate, in particularly MSG, and at least one compound of formula one.

In a more particular embodiment compositions of the present invention contain at least one salt of glutamate, in particularly MSG, at least one compound of formula (I) and at least one purine nucleotide, in particularly inosine monophosphate (IMP) and guanosine monophosphate (GMP).

Compounds of formula (I) may be used in compositions at a concentration of up to 99.9%, particularly 1.0 - 99%, and more particularly 5% - 99%.

In yet a further aspect of the present invention there is provided a method of enhancing or modifying the umami taste and savoury flavour of a consumable product, comprising at least one umami tastant, comprising the step of adding to the said consumable product a compound of formula (I).

Consumable products as used herein means any product that is intended to be placed in the mouth and ingested, or used in the mouth and then discarded. Suitable consumable products include, but are not limited to, food products, beverage products, nutraceutical products, and dental care products including mouth wash.

In yet another aspect of the present invention there is provided a consumable product comprising at least one umami tastant and a compound of formula (I). In a particular embodiment the consumable product is a food or beverage product.

Example food products include, but are not limited to, condiments e.g. sauces, dips, seasoning and the like , savoury products, cereal products, rice products, pasta products, ravioli, tapioca products, sago products, baker's products, biscuit products, pastry products, bread products, confectionery products, dessert products, honey products, treacle products, yeast products, mustard products, vinegar products, processed foods, cooked fruits and vegetable products, meat and meat products, meat analogues/substitutes, jellies, jams, fruit sauces, egg products, dairy products, cheese products, dairy substitute products, soy products, edible oils and fat products,

Example savoury products include, but are not limited to, salty snacks (potato chips, crisps, nuts, tortilla-tostada, pretzels, cheese snacks, corn snacks, potato-snacks, ready-to-eat popcorn, microwaveable popcorn, pork rinds, nuts, crackers, cracker snacks, breakfast cereals, meats, aspic, cured meats (ham, bacon), luncheon/breakfast meats (hotdogs, cold cuts, sausage), tomato products, margarine, peanut butter, soup (clear, canned, cream, instant, UHT),canned vegetables, pasta sauces.

Example beverage products include, but are not limited to, juices, fruit juices, vegetable juices, carbonated soft drinks, beer, wine, hot chocolate, tea and coffee.

In a more particular embodiment the consumable product is a condiment.

Example condiment products include, but are not limited to sauces (cold, warm, instant, preserved, sate, tomato, BBQ Sauce, Ketchup, mayonnaise, salad cream, bechamel), gravy, chutney, salad dressings (shelf stable, refrigerated), dry spice or seasoning compositions, liquid spice or seasoning compositions including pesto and marinades.

Compounds of formula (I), or compositions containing compounds of formula (I) can be added to consumable products by using conventional techniques to directly admix said compounds or said compositions into the consumable product.

The quantities in which compounds of formula (I) may be employed in consumable products may vary within wide limits and depend, inter alia, on the nature of the consumable product, on the effect desired, and on the nature and quantity of any other components, for example other umami tastants or umami and savoury flavour enhancers, in the consumable product. It is well within the purview of the person skilled in the art to decide on suitable quantities of compounds of formula (I) to incorporate into a consumable product depending on the end use and effect required.

Typical, non limiting, concentrations of compounds of formula (I) in consumable products are 0.1 to 5000 ppm, particularly 1.0 - 500ppm, and more particularly 5.0 - 100ppm.

It has also been found that adding mineral salts to the aforementioned compositons and/or consumable products further enhances the umami taste and savoury flavour of said compositions and/or consumable products.

In a further aspect of the present invention there is provided a composition or consumable product comprising at least one umami tastant, at least one compound of formula (I) as herinabove defined, and at least one mineral salt.

The term "mineral salt" as used herein includes salts of sodium, potassium, magnesium, calcium, ammonium, and iron/ferrum, including but not limited to monovalent and bivalent salts.

Non limiting examples of mineral salts include sodium chloride (NaCl), potassium chloride (KCl), magnesium chloride ($MgCl_2$), sea salt, fleur de sel, calcium fluoride ($CaF_2$), calcium dihydrogen phosphate ($Ca(H_2PO_4)_2$), calcium hydrogen phosphate ($CaHPO_4$), tricalcium phosphate ($Ca_3(PO_4)_2$), calcium sulfate ($CaSO_4$),monopotassium phosphate ($KH_2PO_4$), dipotassium phosphate ($K_2HPO_4$), tripotassium phosphate ($K_3PO_4$), magnesium sulphate ($MgSO_4$), ammonium sulphate $(NH_4)_2SO_4$, potassium sulphate ($K_2SO_4$), monosodium dihydrogen phosphate ($NaH_2PO_4$), disodium hydrogen phosphate ($Na_2HPO_4$), trisodium phosphate ($Na_3PO_4$), sodium sulfate ($Na_2SO_4$), ferric pyrophosphate, magnesium phosphate monobasic ($Mg(H_2PO_4)_2$), magnesium phosphate dibasic ($MgHPO_4$), magnesium phosphate tribasic ($Mg_3(PO_4)_2$), silicon dioxide/silica.

The quantities in which mineral salts may be added to said compositions and/or consumable products may vary within wide limits and depend, inter alia, on the nature of the consumable product, on the effect desired, and on the nature and quantity of any other components, for example other umami tastants or umami taste and savoury flavour enhancers such as MSG, in the composition or consumable product. It is well within the purview of the person skilled in the art to decide on suitable quantities of mineral salts to incorporate into a consumable product depending on the end use and effect required.

Typical non limiting, concentrations of mineral salts in consumable products are 0.1 to 8000 ppm.

In a particular embodiment the mineral salts are selected from the group consisting of NaCl, $K_2HPO_4$, and $MgCl_2$.

It has been found that some of the umami taste and savoury flavour enhancing compounds of formula (I) are novel. Therefore in yet a further aspect of the current invention there is provided 1-acetoxy-2-hydroxy-4-oxo-n-octadeca-12-en.

1-acetoxy-2-hydroxy-4-oxo-n-octadeca-12-en occurs naturally and can be isolated from botanical materials, for example from avocado. An example extraction process is provided in example 1.

There now follows a series of non limiting examples that serve to illustrate the invention.

Unless otherwise indicated, percentages are given as wt/wt.

Example 1

Preparation of acetonitrile (hereinafter "ACN") fraction and isolation of compounds of formula (I) from heated avocado pulp.

Avocados (Persea americana MILL. cv. HASS) were purchased from a local trader.
ACN was HPLC grade, water was Millipore grade.

Ethanol, formic acid and sodium L-glutamate (Merck KGA, Darmstadt, Germany). Sodium chloride and maltodextrin came from Sigma-Aldrich (Steinheim, Germany), the yeast extract "Gistex XII LS Pulver AGGL" from DSM Food Specialties Savoury Ingredients (Delft, Nederland).

Heating process:

300 gram pulp of three fresh ripe Hass avocados, separated from peels and seeds, were mashed in a mixer for 30 seconds at 3500 rpm, put into a beaker (500 mL volume) and covered with aluminum foil to ensure that no water/ steam could leave the sample. The sample was heated in a drying oven for 180 mins at 120°C. The temperature was controlled by a thermometer. After heating, the heated pulp was cooled down to room temperature.

Extraction process:

The pulp was transferred to a round-bottom flask (1000mL volume), and extracted with 200mL of pentane for 15min by means of an ultra sonic bath. Using a paper filter, the pentane layer was separated from insoluble residue. The extraction of insoluble residue with pentane was repeated five times. The combined pentane layers were moved in a separatory funnel (2000mL volume), extracted three times with 300mL ethanol/water (8/2;v/v) and extracted two times with 300mL ethanol/water (9/1;v/v) mixture. The combined ethanol/water fractions were freed from ethanol and water by vacuum. The oily residue was dissolved in 300mL ACN.

Removal of Triglycerides by Solid Phase Extraction (SPE):

Separations with SPE were performed on four 100×4.6mm (57μm, 70A) "Strata C18-E" cartridge columns (Phenomenex, Aschaffenburg, Germany) which were conditioned with ACN.

The ACN fraction, split into four aliquots, was applied onto the top of the SPE cartridges. The elution of every cartridge followed with 150ml ACN. The volume of the combined eluents was reduced to 50ml by applying a vacuum. The cleaned fraction obtained (hereinafter "ACN fraction") was separated into 18 fractions by preparative HPLC, the corresponding compounds were isolated and identified by means of LC-MS/MS and NMR spectroscopy.

Preparative High Performance Liquid Chromatography (HPLC):

The preparative HPLC system (Varian, Darmstadt, Germany) consisted of two pumps (ProStar 210), a gradient mixer (1000μL), a Rheodyne injector with a 1900μl loop, an UV/VIS detector (ProStar 325) and an evaporative light scattering detector (ELSD) ("Sedex85" - Sedere, Alfortville Cedex, France). A splitter between the UV/VIS and the ELSD ensured that a flow intensity of 1.0mL/min arrived the ELSD detector.
Separations were performed on a 250×21.2mm, 5μm, "HyperClone" ODS C18 column (Phenomenex, Aschaffenburg, Germany) with a flow rate of 20.0mL/min and 1.9 mL injection volume controlled with the HPLC "Star Chromatography Workstation, Version 6.2" software (Varian, Darmstadt, Germany). Compounds were detected at a wavelength of 220nm and ELSD-Gain 5.
HPLC-Gradient (75.0min): using water (pH5.0, adjusted with 1.0% HCOOH in water) as solvent A, and ACN as solvent B, chromatography was performed starting with solvent A (40%), after 2.5min solvent B (60%) was increased to 75% at 62.5min and to 100% at 65.0min, until 70 minutes. After 70.0min solvent B was reduced to 60% at 72 min, and was held at 60% for 3.0 min until reaching 75 min.

The following compounds were isolated and identified.

1-acetoxy-2,4-dihydroxy-n-heptadeca-16-yne
1-acetoxy-2-hydroxy-4-oxo-n-heptadeca-16-en
1-acetoxy-2,4-dihydroxy-n-heptadeca-16-en
1-acetoxy-2-hydroxy-4-oxo-n-heptadecan
(E,Z,Z)-1-acetoxy-2-hydroxy-4-oxo-heneicosa-5,12,15-trien
(Z,Z)-1-acetoxy-2,4-dihydroxyheneicosa-12,15-dien
(Z,Z)-1-acetoxy-2-hydroxy-4-oxo-heneicosa-12,15-dien

**1-acetoxy-2-hydroxy-4-oxo-n-octadeca-12-en**

MS (APCI$^+$) *m/z* (%): 287 (75, $[M-3H_2O+H]^+$), 305 (80, $[M-2H_2O+H]^+$), 323 (100, $[M-H_2O+H]^+$), 341 (25, $[M+H]^+$), 663 (95, $[2M-H_2O+H]^+$), 681 (40, $[2M+H]^+$); MS (APCI$^-$) *m/z* (%): 339 (40, $[M-H]^-$), 385 (95, $[M+formiate]^-$), 679 (100, $[2M-H]^-$); EPI (APCI$^+$, DP: +50, CE: +30, CES: +25) *m/z* (%): 287 (45, $[M-3H_2O+H]^+$), 305 (50, $[M-2H_2O+H]^+$), 323 (100, $[M-H_2O+H]^+$), 341 (15, $[M+H]^+$); EPI (APCI$^-$, DP: -50, CE: -30, CES: -25) *m/z* (%): 303 (100, $[M-2H_2O-H]^-$), 321 (80, $[M-H_2O-H]^-$), 339 (35, $[M-H]^-$) ; $^1$H NMR (400 MHz, $CDCl_3$), δ/ppm 0.89 [t, 3H, *J* = 6.5 Hz, *J* = 13.1 Hz, H-C(18)], 1.30 [m, 14H, H-C(7), H-C(8), H-C(9), H-C(10), H-C(15), H-C(16), H-C(17)], 1.49 [m, 2H, H-C(6)], 2.03 [dd, 4H, *J* = 6.6 Hz, 13.3 Hz, H-C(11), H-C(14)], 2.11 [s, 3H, H-C(2')], 2.52 [t, 2H, *J* = 5.8 Hz, 12.8 Hz, H-C(5)], 2.79 [m, 2H, H-C(3)], 4.12 [m, 2H, H-C(1)], 4.32 [m, 1H, H-C(2)], 5.37 [m, 4H, H-C(12), H-C(13)]; $^{13}$C NMR (400 MHz, $CDCl_3$), δ/ppm 14.1 [C-18], 20.9 [C-2'], 22.6 [C-17], 25.3 [C-6], 27.2 [C-11, C-14], 29.1 [C-7], 29.3 [C-8], 29.4 [C-15], 29.6 [C-9], 29.7 [C-10], 31.5 [C-16], 41.2 [C-5], 43.0 [C-3], 68.6 [C-2], 70.8 [C-1], 130.2 [C-12, C-13], 171.3 [C-1'], 210.9 [C-4]. The coupling constant J = 11.69 Hz of the signals at δH = 5.35 ppm and δH = 5.37 ppm indicates (Z)-configuration of the two double bonds in the molecule.

Example 2

Sensory analysis of the umami enhancing thresholds of the compounds isolated from the ACN fraction of heated avocado pulp as described in example 1.

Water for sensory evaluation was commercially available table water ("Evian®", Danone, Wiesbaden, Germany).

"Model Broth" contained monosodium L-glutamate monohydrate (MSG) (1.9g), maltodextrin (6.375g), sodium chloride (2.9g) and yeast extract (2.1g), in 1000 mL of water.

An ACN fraction from avocado pulp was prepared and the following compounds were isolated as described in example 1 herein-above: 1-acetoxy-2-hydroxy-4-oxo-n-heneicosa-5,12,15-trien; 1-acetoxy-2,4-dihydroxy-n-heneicosa-12,15-dien; 1-acetoxy-2,4-dihydroxy-n-heptadeca-16-yne, 1-acetoxy-2-hydroxy-4-keto-n-octadeca-12-en; 1-acetoxy-2,4-dihydroxy-n-heptadeca-16-en; 1-acetoxy-2-hydroxy-4-oxo-n-heneicosa-12,15-dien; 1-acetoxy-2-hydroxy-4-keto-n-heptadeca-16-en; 1-acetoxy-2-hydroxy-4-keto-n-heptadecane.

25mg of each of the compounds listed hereinabove were separately dissolved in 0.2mL ethanol, then 20.0mL of model broth was added to each sample. Each sample was then diluted with model broth in 1:1 (v/v) steps, until sets of nine solutions with dilution factors of 1, 2, 4, 8,16, 32, 64, 128, and 512 were obtained for each compound.

The samples were evaluated by a panel of trained experts. The following sensorial evaluation procedure was followed for each set of samples containing a different compound of formula (I).

In a way that avoided any mixing, 1 mL of one of the sample solutions was applied to the left part of each panelists tongue, and 1 mL of Model Broth as a blank value/neutral reference was applied to the right part of the tongue. The panelists were asked to compare the taste on both halves of their tongues. After 5 to 30 seconds the panelists were then asked to rub their tongues against the roof of their mouths. Panelists were then asked if they could distinguish the sample from the blank (Model Broth).
After each sample, the oral cavity was rinsed with water and a break of 5 to 10 minutes was observed until the stimulus completely disappeared.

Multiple sample solutions were tested in this way, one after the other in ascending order of concentration, to determine the weakest concentration at which the compounds of formula (I) enhanced the umami taste of the model broth.

The calculation of the umami enhancement threshold was then performed as described in the literature (ASU: L 00.90-9, 1999), via the geometric mean.

By "umami enhancement threshold" is meant, the mean concentration at which a compound enhances the umami taste of a model broth.

For each panelist, the following formula was used to determine the mean umami enhancement threshold:

$$E = \sqrt{c_e \times c_{e-1}}$$

wherein $c_e$ is the concentration (in ppm) of the weakest concentration identified as having an umami enhancing effect, $C_{e-1}$ is the concentration (in ppm) of the anteceding sample solution and E is the mean umami enhancement threshold.

To calculate the mean umami enhancement threshold for the panel, the following formula was used:

$$\overline{E} = \sqrt[n]{\prod_{i=1}^{n} E_i}$$

Wherein n is the number of panelists, $E_i$ is the product of the umami enhancement thresholds calculated for each panelist as specified above.

The panel calculated mean umami enhancement threshold for each compound is indicated in table 1 below.

Table 1

| Compounds | Umami enhancement threshold concentration in "Model Broth" as calculated for the panel ppm |
|---|---|
| 1-acetoxy-2-hydroxy-4-oxo-n-heneicosa-5,12,15-trien | 0.7 |
| 1-acetoxy-2,4-dihydroxy-n-heneicosa-12,15-dien | 0.7 |
| 1-acetoxy -2,4-dihydroxy-n-heptadeca-16-yne | 1.5 |
| 1-acetoxy-2-hydroxy-4-keto-n-octadeca-12-en | 1.8 |
| 1-acetoxy-2,4-dihydroxy-n-heptadeca-16-en | 2.8 |
| 1-acetoxy-2-hydroxy-4-oxo-n-heneicosa-12,15-dien | 3.2 |
| 1-acetoxy-2-hydroxy-4-keto-n-heptadeca-16-en | 3.5 |
| 1-acetoxy-2-hydroxy-4-keto-n-heptadecane | 5.6 |

As can be seen in table 1 compounds of the present invention displayed an umami enhancement threshold within the range from 0.7ppm to 5.6ppm.

## Claims

1. A method of enhancing the umami taste and savoury flavour of a composition comprising at least one umami tastant, comprising the step of adding to the aforementioned composition at least one compound of formula (I)

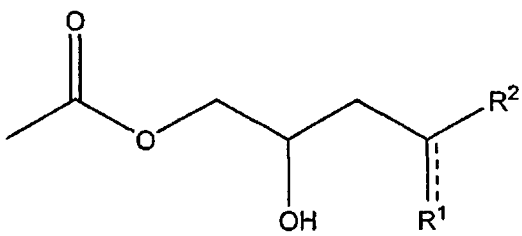

(I)

wherein $R^1$ is selected from O and OH, the dotted line representing a bond present when $R^1$ is O
$R^2$ is a hydrocarbon residue having 6 to 22 carbon atoms, comprising from 0-4 unsaturated carbon-carbon bonds.

2. A method according to claims 1, comprising the step of adding to the aforementioned composition at least one compound of formula (I) selected from the group consisting of: 1-acetoxy-2-hydroxy-4-oxo-n-heneicosa-5,12,15-trien; 1-acetoxy-2,4-dihydroxy-n-heneicosa-12, 15-dien; 1-acetoxy-2,4-dihydroxy-n-heptadeca-16-yne; 1-acetoxy-2-hydroxy-4-keto-n-octadeca-12-en; 1-acetoxy-2,4-dihydroxy-n-heptadeca-16-en; 1-acetoxy-2-hydroxy-4-oxo-n-heneicosa-12,15-dien; 1-acetoxy-2-hydroxy-4-keto-n-heptadeca-16-en; 1-acetoxy-2-hydroxy-4-keto-n-heptadecane.

3. A method of enhancing or modifying the umami taste and savoury flavour of a consumable product, comprising at least one umami tastant, comprising the step of adding to said consumable product a compound of formula (I) as defined in claims 1 and 2.

4. A composition comprising at least one umami tastant and at least one compound of formula (I) as defined in claims 1 and 2.

5. A consumable product comprising at least one umami tastant and a compound of formula (I) as defined in claims 1 and 2.

6. A composition or consumable product according to claims 4 and 5 further comprising monosodium glutamate (MSG).

7. A composition or consumable product according to claims 4 to 6 further comprising inosine monophosphate (IMP) and guanosine monophosphate (GMP).

8. A consumable product according to claims 5 to 7 wherein the consumable product is a food or beverage product.

9. A consumable product according to claim 8 wherein the consumable product is a condiment.

10. A composition or consumable product according to claims 4 to 9 further comprising a mineral salt.

11. A composition or consumable product according to claim 10 wherein the mineral salt is selected from the group consisting of NaCl, $K_2HPO_4$, $MgCl_2$

12. 1-acetoxy-2-hydroxy-4-oxo-n-octadeca-12-en.

13. Use of a compound of formula (I) as defined in claims 1 and 2 as an umami taste and savoury flavour enhancer.

## Patentansprüche

1. Verfahren zum Verstärken des Umamigeschmacks und pikanten Aromas einer Zusammensetzung, die wenigstens einen Umami-Geschmacksstoff umfasst, umfassend den Schritt des Zugebens wenigstens einer Verbindung der Formel (I)

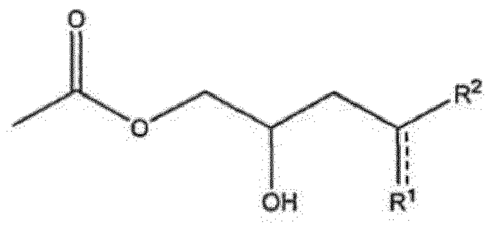

(I)

wobei $R^1$ ausgewählt ist aus 0 und OH und die gestrichelte Linie eine Bindung darstellt, die vorhanden ist, wenn $R^1$ 0 ist,
$R^2$ ein Kohlenwasserstoffrest mit 6 bis 22 Kohlenstoffatomen ist, umfassend 0-4 nichtgesättigte Kohlenstoff-Kohlenstoff-Bindungen,

zu der genannten Zusammensetzung.

2. Verfahren gemäß Anspruch 1, umfassend den Schritt des Zugebens wenigstens einer Verbindung der Formel (I), ausgewählt aus der Gruppe bestehend aus: 1-Acetoxy-2-hydroxy-4-oxo-n-heneicosa-5,12-15-trien; 1-Acetoxy-2,4-dihydroxy-n-heneicosa-12,15-dien; 1-Acetoxy-2,4-dihydroxy-n-heptadeca-16-in; 1-Acetoxy-2-hydroxy-4-keto-n-octadeca-12-en; 1-Acetoxy-2,4-dihydroxy-n-heptadeca-16-en; 1-Acetoxy-2-hydroxy-4-oxo-n-heneicosa-12,15-dien, 1-Acetoxy-2-hydroxy-4-keto-n-heptadeca-16-en; 1-Acetoxy-2-hydroxy-4-keto-heptadecan, zu der genannten Zusammensetzung.

3. Verfahren zum Verstärken oder Modifizieren des Umamigeschmacks und des pikanten Aromas eines verzehrbaren Produkts, das wenigstens einen Umami-Geschmacksstoff umfasst, umfassend den Schritt des Zugebens einer Verbindung der Formel (I) gemäß Ansprüchen 1 und 2 zu dem verzehrbaren Produkt.

4. Zusammensetzung, umfassend wenigstens einen Umami-Geschmacksstoff und wenigstens eine Verbindung der Formel (I) gemäß Ansprüchen 1 und 2.

5. Verzehrbares Produkt, umfassend wenigstens einen Umami-Geschmacksstoff und eine Verbindung der Formel (I) gemäß Ansprüchen 1 und 2.

6. Zusammensetzung oder verzehrbares Produkt gemäß Ansprüchen 4 und 5, ferner umfassend Mononatriumglutamat (MSG).

7. Zusammensetzung oder verzehrbares Produkt gemäß Ansprüchen 4 bis 6, ferner umfassend Inosinmonophosphat (IMP) und Guanosinmonophosphat (GMP).

8. Verzehrbares Produkt gemäß Ansprüchen 5 bis 7, wobei das verzehrbare Produkt ein Lebensmittel- oder Getränkeprodukt ist.

9. Verzehrbares Produkt gemäß Anspruch 8, wobei das verzehrbare Produkt ein Gewürz ist.

10. Zusammensetzung oder verzehrbares Produkt gemäß Ansprüchen 4 bis 9, ferner umfassend ein Mineralsalz.

11. Zusammensetzung oder verzehrbares Produkt gemäß Anspruch 10, wobei das Mineralsalz ausgewählt ist aus der Gruppe bestehend aus NaCl, $K_2HPO_4$, $MgCl_2$.

12. 1-Acetoxy-2-hydroxy-4-oxo-n-octadeca-12-en.

13. Verwendung einer Verbindung der Formel (I) gemäß Ansprüchen 1 und 2 als Verstärker von Umamigeschmack und pikantem Aroma.

## Revendications

1. Méthode pour améliorer le goût umami et la saveur salée d’une composition comprenant au moins un agent de sapidité umami, comprenant l’étape consistant à ajouter à la composition susmentionnée au moins un composé de formule (I)

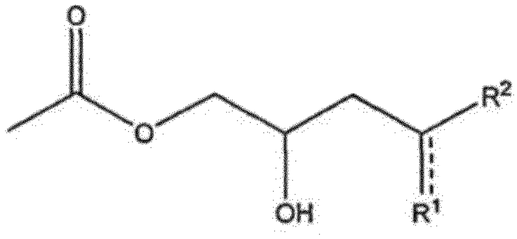

(I)

dans laquelle $R^1$ est choisi parmi 0 et OH, la ligne en pointillés représentant une liaison présente lorsque $R^1$ est 0 $R^2$ est un résidu hydrocarboné ayant de 6 à 22 atomes de carbone, comprenant de 0 à 4 liaisons carbone-carbone insaturées.

**2.** Méthode selon la revendication 1, comprenant l'étape consistant à ajouter à la composition susmentionnée au moins un composé de formule (I) choisi dans le groupe constitué par : le 1-acétoxy-2-hydroxy-4-oxo-n-héneicosa-5,12,15-triène ; le 1-acétoxy-2,4-dihydroxy-n-héneicosa-12,15-diène ; le 1-acétoxy-2,4-dihydroxy-n-heptadéca-16-yne ; le 1-acétoxy-2-hydroxy-4-céto-n-octadéca-12-ène ; le 1-acétoxy-2,4-dihydroxy-n-heptadéca-16-ène ; le 1-acétoxy-2-hydroxy-4-oxo-n-héneicosa-12,15-diène; le 1-acétoxy-2-hydroxy-4-céto-n-heptadéca-16-ène ; le 1-acétoxy-2-hydroxy-4-céto-n-heptadécane.

**3.** Méthode pour améliorer ou modifier le goût umami et la saveur salée d'un produit comestible, comprenant au moins un agent de sapidité umami, comprenant l'étape consistant à ajouter audit produit comestible un composé de formule (I) tel que défini dans les revendications 1 et 2.

**4.** Composition comprenant au moins un agent de sapidité umami et au moins un composé de formule (I) tel que défini dans les revendications 1 et 2.

**5.** Produit comestible comprenant au moins un agent de sapidité umami et un composé de formule (I) tel que défini dans les revendications 1 et 2.

**6.** Composition ou produit comestible selon les revendications 4 et 5, comprenant en outre du glutamate monosodique (GMS).

**7.** Composition ou produit comestible selon les revendications 4 à 6 comprenant en outre de l'inosine monophosphate (IMP) et de la guanosine monophosphate (GMP).

**8.** Produit comestible selon les revendications 5 à 7, **caractérisé en ce que** le produit comestible est un aliment ou une boisson.

**9.** Produit comestible selon la revendication 8, **caractérisé en ce que** le produit comestible est un condiment.

**10.** Composition ou produit comestible selon les revendications 4 à 9, comprenant en outre un sel minéral.

**11.** Composition ou produit comestible selon la revendication 10, **caractérisé en ce que** le sel minéral est choisi dans le groupe constitué par le NaCl, le $K_2HPO_4$, le $MgCl_2$.

**12.** 1-acétoxy-2-hydroxy-4-oxo-n-octadéca-12-ène.

**13.** Utilisation d'un composé de formule (I) tel que défini dans les revendications 1 et 2 en tant qu'agent améliorant le goût umami et la saveur salée.

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Non-patent literature cited in the description


- Chemicals Used in Food Processing. National Academy of Sciences, vol. 1274, 63-258
- *Perfume and Flavour Materials of Natural Origin,* 1960
- Perfume and Flavour Chemicals. Allured Publishing Corporation, 1994, vol. I, II
- Flavourings. Wiley-VCH, 1998
- CTFA Cosmetic Ingredient Handbook
- Handbook of Industrial Chemical Additives. 2000